(19) Europäisches Patentamt / European Patent Office / Office européen des brevets

(11) **EP 4 434 439 A1**

(12) **EUROPEAN PATENT APPLICATION**

(43) Date of publication:
**25.09.2024 Bulletin 2024/39**

(21) Application number: **24164205.7**

(22) Date of filing: **18.03.2024**

(51) International Patent Classification (IPC):
**A61B 3/113** $^{(2006.01)}$   **G02B 27/00** $^{(2006.01)}$
**G02B 27/01** $^{(2006.01)}$   **G06F 3/01** $^{(2006.01)}$

(52) Cooperative Patent Classification (CPC):
**A61B 3/113; G02B 27/0093; G02B 27/017;
G06F 3/013;** G02B 2027/0138; G02B 2027/0178

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **21.03.2023 SE 2350314**

(71) Applicant: **Tobii AB**
**182 17 Danderyd (SE)**

(72) Inventors:
• **Johansson, Simon**
**182 17 Danderyd (SE)**

• **Rosell, Mikael**
**182 17 Danderyd (SE)**
• **Ljungzell, Erik**
**182 17 Danderyd (SE)**
• **Lundahl, Rickard**
**182 17 Danderyd (SE)**
• **Rana, Pravin Kumar**
**182 17 Danderyd (SE)**
• **Keshavdas, Shanker**
**18217 Danderyd (SE)**
• **Zamani, Neda**
**18217 Danderyd (SE)**
• **Wu, Hanwei**
**18217 Danderyd (SE)**

(54) **AN EYE TRACKING SYSTEM AND METHODS OF USING AN EYE TRACKING SYSTEM**

(57) A method for determining a gaze convergence function for a user of an eye tracking system. The method comprising presenting a first calibration object to the user on one or more display screens, wherein the first calibration object appears to be at a known first object distance from the user. While the first calibration object is being presented, the method: captures a calibration images of the user's left eye and right eye and calculates a first interpupillary distance, IPD, value as the distance between: a first left pupil centre value as a 3D coordinate; and a first right pupil centre value as a 3D coordinate. The method performs similar processing steps for a second calibration object to calculate a second IPD value as the distance between a second left pupil centre 3D value and a second right pupil centre 3D value. The method then determines a gaze convergence function which defines a relationship between IPD values and gaze convergence distances based on: i) the first IPD value and the associated first object distance; and ii) the second IPD value and the associated second object distance.

Figure 6

## Description

## Field

The present disclosure generally relates to the field of eye tracking. In particular, the present disclosure relates to methods and systems for determining a gaze convergence function for a user of an eye tracking system. The present disclosure also relates to determining a gaze convergence distance based on a gaze convergence function for a user of an eye tracking system.

## Background

In eye tracking applications, digital images are retrieved of the eyes of a user and the digital images are analysed in order to estimate gaze direction of the user. The estimation of the gaze direction may be based on computer-based image analysis of features of the imaged eye. One known example method of eye tracking includes the use of infrared light and an image sensor. The infrared light is directed towards eye(s) of a user and the reflection of the light is captured by an image sensor.

Portable or wearable eye tracking devices have been previously described. One such eye tracking system is described in U.S. Pat. No. 9,041,787 and PCT patent publication number WO 2019/158709 (which are hereby incorporated by reference in their entirety). A wearable eye tracking device is described using illuminators and cameras for determining gaze direction.

## Summary

According to a first aspect of the disclosure, there is provided a method for determining a gaze convergence function for a user of an eye tracking system, the method comprising:

> presenting a first calibration object to the user on one or more display screens, wherein the first calibration object appears to be at a known first object distance from the user;
> while the first calibration object is being presented:
>
>> capturing a calibration image of the user's left eye and determining a first left pupil centre value, which represents the centre of the pupil of the user's left eye as a 3-dimensional coordinate, 3D, based on the captured calibration image of the user's left eye;
>> capturing a calibration image of the user's right eye and determining a first right pupil centre value, which represents the centre of the pupil of the user's right eye as a 3-dimensional coordinate, based on the captured calibration image of the user's right eye;
>
> calculating a first interpupillary distance, IPD, value as the distance between the first left pupil centre value and the first right pupil centre value;
presenting a second calibration object to the user on one or more display screens, wherein the second calibration object appears to be at a known second object distance from the user;
while the second calibration object is being presented:

> capturing a calibration image of the user's left eye and determining a second left pupil centre value, which represents the centre of the pupil of the user's left eye as a 3-dimensional coordinate, based on the captured calibration image of the user's left eye;
> capturing a calibration image of the user's right eye and determining a second right pupil centre value, which represents the centre of the pupil of the user's right eye as a 3-dimensional coordinate, based on the captured calibration image of the user's right eye;

calculating a second IPD value as the distance between the second left pupil centre value and the second right pupil centre value;
determining a gaze convergence function which defines a relationship between IPD values and gaze convergence distances based on: i) the first IPD value and the associated first object distance; and ii) the second IPD value and the associated second object distance.

It is advantageous to use 3-dimensional coordinates for the centres of the pupils when calculating the IPD that is used for determining the gaze convergence function because any movement of the eye tracking system relative to the user's eyes does not affect the calculation of the IPD.

The method may further comprise:

> presenting one or more further calibration objects to the user on one or more display screens, wherein each of the one or more further calibration objects appears to be at a known further object distance from the user;
> while each of the further calibration objects is being presented:
>
>> capturing a calibration image of the user's left eye and determining a further left pupil centre value, which represents the centre of the pupil of the user's left eye as a 3-dimensional coordinate, based on the captured calibration image of the user's left eye;
>> capturing a calibration image of the user's right eye and determining a further right pupil centre value, which represents the centre of the pupil of the user's right eye as a 3-dimensional coordinate, based on the captured calibration image of the user's right eye;

calculating a further IPD value for each of the further calibration objects as the distance between the further first left pupil centre value and the further first right pupil centre value for the calibration images that were captured with the respective one or more further calibration objects being presented; and determining the gaze convergence function also based on, for each of the one or more further calibration objects: i) the further IPD value and the associated further object distance.

The gaze convergence function may defines a linear relationship between: i) the inverse of IPD values; and ii) the inverse of gaze convergence distances.

Capturing a calibration image of the user's eye and determining a pupil centre value may comprise: simultaneously capturing a first calibration image and a second calibration image of the user's eye from different cameras and determining the pupil centre value, which represents the centre of the pupil of the user's eye as a 3-dimensional coordinate, based on the first and second calibration images.

The method may further comprise: while the calibration objects are being presented:

illuminating the user's left eye with a plurality of illuminators and capturing a calibration image of the user's left eye that includes reflections of light provided by the plurality of illuminators as a plurality of glints, determining a 3-dimensional position of the cornea of the user's left eye based on the plurality of glints, and determining the left pupil centre value based on: i) the captured calibration image of the user's left eye; and ii) the determined 3-dimensional position of the cornea of the user's left eye;

illuminating the user's right eye with a plurality of illuminators and capturing a calibration image of the user's right eye that includes reflections of light provided by the plurality of illuminators as a plurality of glints, determining a 3-dimensional position of the cornea of the user's right eye based on the plurality of glints, and determining the right pupil centre value based on: i) the captured calibration image of the user's right eye; and ii) the determined 3-dimensional position of the cornea of the user's right eye.

The method may further comprise:

while the first calibration object is being presented, determining a first gaze angle for the user; while the second calibration object is being presented, determining a second gaze angle for the user; and wherein determining the gaze convergence function is based on: i) the first IPD value, the associated first object distance, and the determined first gaze angle; and ii) the second IPD value, the associated second object distance, and the determined second gaze angle.

The gaze convergence function may define the gaze convergence distance as a function of IPD value and gaze angle.

The method may further comprise: determining a plurality of gaze convergence functions, each gaze convergence function being applicable for different gaze angles, wherein each of the gaze convergence functions defines the gaze convergence distance as a function of IPD value.

Each of the gaze convergence functions may be applicable for a different range of gaze angles. Optionally, the range of gaze angles may be offset from each other in a horizontal plane and / or a vertical plane.

The method may further comprise: determining a plurality of gaze convergence functions, each gaze convergence function being applicable for a different gaze zone, wherein each of the gaze convergence functions defines the gaze convergence distance as a function of IPD value.

The eye tracking system may be a head-mounted eye tracking system.

There is also disclosed a method of determining a gaze convergence distance for a user of an eye tracking system, the method comprising:

capturing an image of the user's left eye and determining a left pupil centre value, which represents the centre of the pupil of the user's left eye as a 3-dimensional coordinate;

capturing an image of the user's right eye and determining a right pupil centre value, which represents the centre of the pupil of the user's right eye as a 3-dimensional coordinate;

calculating an interpupillary distance, IPD, value as the distance between the left pupil centre value and the right pupil centre value;

applying a gaze convergence function to the calculated IPD value to determine a gaze convergence distance, wherein the gaze convergence function defines a relationship between IPD values and gaze convergence distances.

The method may further comprise: using the determined gaze convergence distance to control the focal length of a varifocal lens of the eye tracking system.

The eye tracking system may be an extended reality system.

The method may further comprise:

presenting a plurality of selectable objects to the user on one or more display screens, wherein the first calibration object, wherein each of the plurality of selectable objects is at a known object distance from the user and are at a known location on the on one or more display screens;

determining a gaze angle for the user at the same time as the images of the user's left and right eyes are captured; and

using the determined gaze convergence distance in combination with the determined gaze angle to identify one of the selectable objects as a selected object.

The method may further comprise determining the gaze convergence function for the user of an eye tracking system, wherein determining the gaze convergence function for the user of an eye tracking system comprises:

presenting a first calibration object to the user on one or more display screens, wherein the first calibration object appears to be at a known first object distance from the user;

while the first calibration object is being presented:

capturing a calibration image of the user's left eye and determining a first left pupil centre value, which represents the centre of the pupil of the user's left eye as a 3-dimensional coordinate, based on the captured calibration image of the user's left eye;

capturing a calibration image of the user's right eye and determining a first right pupil centre value, which represents the centre of the pupil of the user's right eye as a 3-dimensional coordinate, based on the captured calibration image of the user's right eye;

calculating a first interpupillary distance, IPD, value as the distance between the first left pupil centre value and the first right pupil centre value;

presenting a second calibration object to the user on one or more display screens, wherein the second calibration object that appears to be at a known second object distance from the user;

while the second calibration object is being presented:

capturing a calibration image of the user's left eye and determining a second left pupil centre value, which represents the centre of the pupil of the user's left eye as a 3-dimensional coordinate, based on the captured calibration image of the user's left eye;

capturing a calibration image of the user's right eye and determining a second right pupil centre value, which represents the centre of the pupil of the user's right eye as a 3-dimensional coordinate, based on the captured calibration image of the user's right eye;

calculating a second IPD value as the distance between the second left pupil centre value and the second right pupil centre value;

determining a gaze convergence function based on: i) the first IPD value and the associated first object distance; and ii) the second IPD value and the associated second object distance.

There is also discussed an eye tracking system that is configured to perform any method disclosed herein.

There may be provided a computer program, which when run on a computer, causes the computer to configure any apparatus, including a controller, device or system disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

The computer program may be provided on a computer readable medium, which may be a physical computer readable medium such as a disc or a memory device, or may be embodied as a transient signal. Such a transient signal may be a network download, including an internet download. There may be provided one or more non-transitory computer-readable storage media storing computer-executable instructions that, when executed by a computing system, causes the computing system to perform any method disclosed herein.

## Brief Description of the Drawings

One or more embodiments will now be described by way of example only with reference to the accompanying drawings in which:

Figure 1 shows a simplified view of an eye tracking system in a head-mounted device;
Figure 2 shows a simplified example of an image of a pair of eyes, captured by an eye tracking system such as the system of Figure 1;
Figure 3 shows a head-mounted device that is mounted on the head of a user;
Figure 4 illustrates various properties associated with a user's eyes when they are looking at an object;
Figure 5 shows a plot of i) the inverse of IPD values (as shown on the horizontal axis); and ii) the inverse of gaze convergence distances (as shown on the vertical axis);
Figure 6 shows an example embodiment of a head-mounted eye tracking system according to the present disclosure;
Figure 7 shows an example embodiment of a method for determining a gaze convergence function for a user of an eye tracking system; and

Figure 8 shows an example embodiment of a method of determining a gaze convergence distance for a user of an eye tracking system.

## Detailed Description

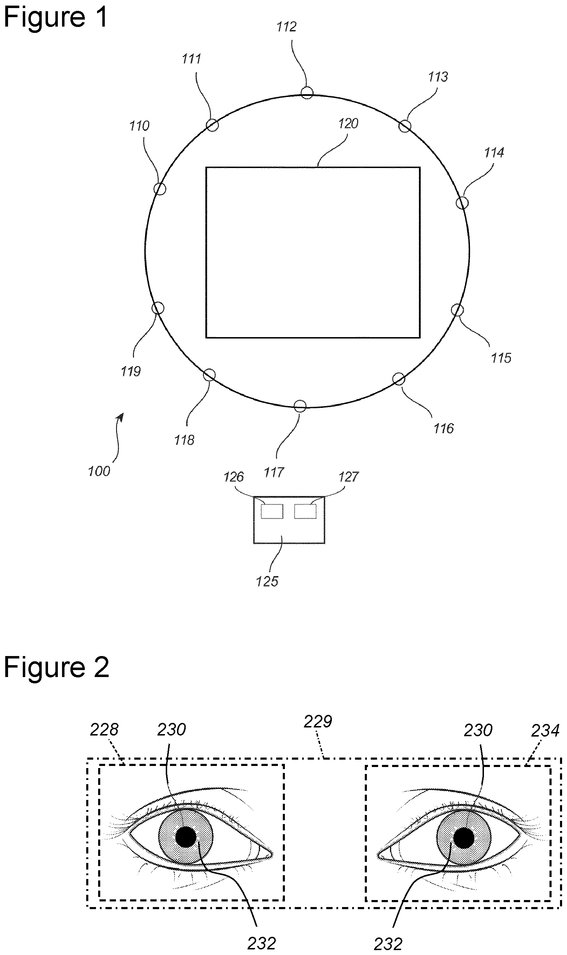

Figure 1 shows a simplified view of an eye tracking system 100 (which may also be referred to as a gaze tracking system) in a head-mounted device in the form of a virtual or augmented reality (VR or AR) device or VR or AR glasses or anything related, such as extended reality (XR) or mixed reality (MR) headsets. The system 100 comprises an image sensor 120 (e.g., a camera) for capturing images of the eyes of the user. The system may optionally include one or more illuminators 110-119 (also referred to herein as light sources) for illuminating the eyes of a user, which may for example be light emitting diodes (LEDs) emitting light in the infrared frequency band, or in the near infrared frequency band and which may be physically arranged in a variety of configurations. The image sensor 120 may for example be an image sensor of any type, such as a complementary metal oxide semiconductor (CMOS) image sensor or a charged coupled device (CCD) image sensor. The image sensor may consist of an integrated circuit containing an array of pixel sensors, each pixel containing a photodetector and an active amplifier. The image sensor may be capable of converting light into digital signals. In one or more examples, it could be an infrared image sensor or IR image sensor, an RGB sensor, an RGBW sensor or an RGB or RGBW sensor with IR filter.

The eye tracking system 100 may comprise circuitry or one or more controllers 125, for example including a receiver 126 and processing circuitry 127, for receiving and processing the images captured by the image sensor 120. The circuitry 125 may for example be connected to the image sensor 120 and the optional one or more illuminators 110-119 via a wired or a wireless connection and be co-located with the image sensor 120 and the one or more illuminators 110-119 or located at a distance, e.g., in a different device. In another example, the circuitry 125 may be provided in one or more stacked layers below the light sensitive surface of the light sensor 120.

The eye tracking system 100 may include a display (not shown) for presenting information and/or visual stimuli to the user. The display may comprise a VR display which presents imagery and substantially blocks the user's view of the real-world or an AR display which presents imagery that is to be perceived as overlaid over the user's view of the real-world.

The location of the image sensor 120 for one eye in such a system 100 is generally away from the line of sight for the user in order not to obscure the display for that eye. This configuration may be, for example, enabled by means of so-called hot mirrors which reflect a portion of the light and allows the rest of the light to pass, e.g., infrared light is reflected, and visible light is allowed to pass.

While in the above example the images of the user's eye are captured by a head-mounted image sensor 120, in other examples the images may be captured by an image sensor that is not head-mounted. Such a non-head-mounted system may be referred to as a remote system.

Figure 2 shows a simplified example of an image 229 of a pair of eyes, captured by an eye tracking system such as the system of Figure 1. The image 229 can be considered as including a right-eye-image 228, of a person's right eye, and a left-eye-image 234, of the person's left eye. In this example the right-eye-image 228 and the left-eye-image 234 are both parts of a larger image of both of the person's eyes. In other examples, separate image sensors may be used to acquire the right-eye-image 228 and the left-eye-image 234. In further still examples, multiple image sensors may be used to acquire images capturing both eyes.

The system may employ image processing (such as digital image processing) for extracting features in the image. The system may for example identify a position of the pupil 230 in the one or more images captured by the image sensor. The system may determine the position of the pupil 230 using a pupil detection process. The system may also identify corneal reflections (also known as glints) 232 located in close proximity to the pupil 230. The system may estimate a corneal centre and / or a distance to the user's eye based on the corneal reflections 232. For example, the system may match each of the individual corneal reflections 232 for each eye with a corresponding illuminator and determine the corneal centre of each eye and / or the distance to the user's eye based on the matching. To a first approximation, the eye tracking system may determine an optical axis of the eye of the user as the vector passing through a centre of the pupil 230 and the corneal centre. The direction of gaze corresponds to the axis from the fovea of the eye through the corneal centre (visual axis). The angle between the optical axis and the gaze direction is the *foveal offset,* which typically varies from user to user and is in the range of a few degrees. The eye tracking system may perform a calibration procedure, instructing the user to gaze in a series of predetermined directions (e.g., via instructions on a screen), to determine the fovea offset. The determination of the optical axis described above is known to those skilled in the art and often referred to as pupil centre corneal reflection (PCCR). PCCR is not discussed in further detail here.

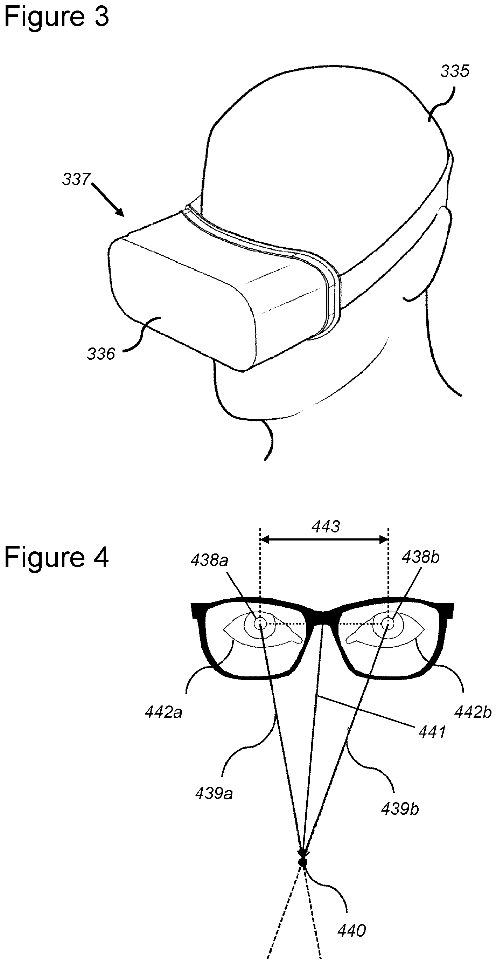

Figure 3 shows a head-mounted device 337 that is mounted on the head of a user 335. The head-mounted device 337 in this example is an extended reality (XR) headset. XR headsets include virtual reality (VR) headsets, augmented reality (AR) headsets and mixed reality (MR) headsets. The head-mounted device 337 includes a 3-dimensional (3D) display screen 336 that is able to visualize objects that appear to be at certain distances from the user in response to a control signal received from a computer. The head-mounted device 337 can often also determine a gaze angle using one or more gaze tracking sensors, as is known in the art.

The 3D display screen 336 may for example be a stereoscopic display screen. Alternatively, the 3D display screen 336 may be a volumetric 3D display screen, being either autostereoscopic or automultiscopic. This indicates that they may create 3D imagery visible to an unaided eye, without requiring stereo goggles or stereo head-mounted displays. Consequently, various of the eye tracking systems that are described herein can be provided as either a head mounted device or a remote system that does not require stereo goggles or stereo head-mounted displays. In a further example, the 3D display screen can be a remote display screen where stereoscopic glasses are needed to visualize the 3D effect to the user.

A stereoscopic scene in a VR headset is rendered at a fixed depth (focal plane) away from the eyes. As a result, users may experience visual fatigue and eye strain when looking at different objects in the scene if the eyes' vergence at that time is not aligned with the focal plane of the stereoscopic scene. This phenomenon is the so-called vergence-accommodation conflict problem.

As will be discussed in detail below, examples disclosed herein can provide an improved method of determining the vergence distance of the user's eyes. The determined vergence distance can then be used to automatically adjust a varifocal VR headset to set the correct focal plane of the stereoscopic scene for the user.

Figure 4 illustrates various properties associated with a user's eyes when they are looking at an object, which will referred to below.

In Figure 4, the user's right eye 442a and the user's left eye 442b are shown. A gaze ray 439a is shown for the user's right eye 442a, which originates from the centre of the user's right pupil 438a. A gaze ray 439b is shown for the user's left eye 442b, which originates from the centre of the user's left pupil 438b. A convergence point 440 can be determined as the point of intersection of the gaze rays 439a, 439b from each eye. A gaze convergence distance is the distance between the user and the convergence point 440. The gaze convergence distance can be calculated as the distance from the left eye 442b to the convergence point 440 (i.e. along the gaze ray 439b for the left eye 442b), or it can be calculated as the distance from the right eye 442a to the convergence point 440 (i.e. along the gaze ray 439a for the right eye 442a), or it can be calculated as the distance 441 from a normal between the left eye 442b and the right eye 442a to the convergence point 440.

The gaze convergence distance can be defined as the distance between the system origin of a VR headset and the intersection of the left and right gaze rays 439a, 439b. Two rays in 3D space only intersect if they are on the same plane, and the probability that two rays in 3D space intersect in practice is very small. Therefore, one way of determining the intersection point of two rays in 3D space is to compute the point between two rays where the two rays are closest. However, this approach is susceptible to noise in the determination of the gaze origin (the cornea centre).

The embodiments that will be described below use a simple relationship between the interpupillary distance (IPD) 443 and the convergence distance 441 (however the convergence distance is defined, as indicated above). The IPD 443 is the distance between the centre of the left pupil 438b and the centre of the right pupil 438a.

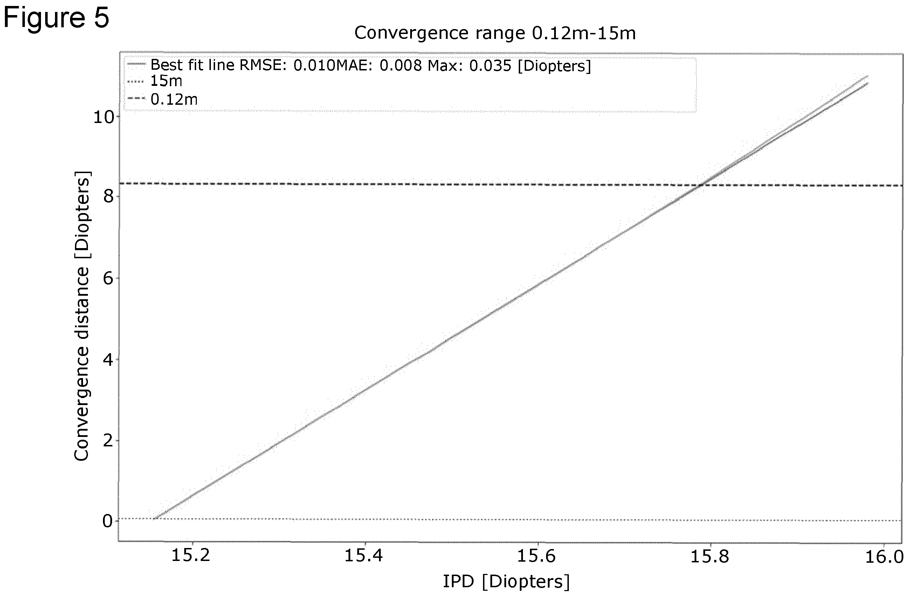

Figure 5 shows that it has been found that, for convergence distances that are relevant to eye tracking systems, there exists a linear relationship between: i) the inverse of IPD values (as shown on the horizontal axis); and ii) the inverse of gaze convergence distances (as shown on the vertical axis). The inverses of such values / distances (i.e. 1/(IPD value) and 1/(gaze convergence distance) are measured in dioptres. Therefore, it is possible to determine a convergence distance by applying a linear function to a measurement of the user's instantaneous IPD value. Furthermore, since a gaze convergence function that defines the relationship between the inverse of the IPD value and the inverse of the gaze convergence distance is linear, it is computationally efficient; both in terms of a calibration operation that will require the linear function to be fitted to calibration values, and also in terms of the subsequent application of the gaze convergence function.

Since each person has different IPD and vergence properties, it can be appropriate to conduct a personal calibration operation to obtain a model (in this example a linear gaze convergence function) between the IPD and the convergence distance for each person. As will now be discussed in more detail, calibration samples can be obtained by displaying stimulus points at multiple depths and angles. The user will then be instructed to look at those stimulus points. Calibration samples can then be collected that include the stimulus depths and the corresponding user IPD measurements. A linear model can then be fitted to the calibration samples (for example a damped least square or other algorithms can be used) to minimise the calibration sample errors.

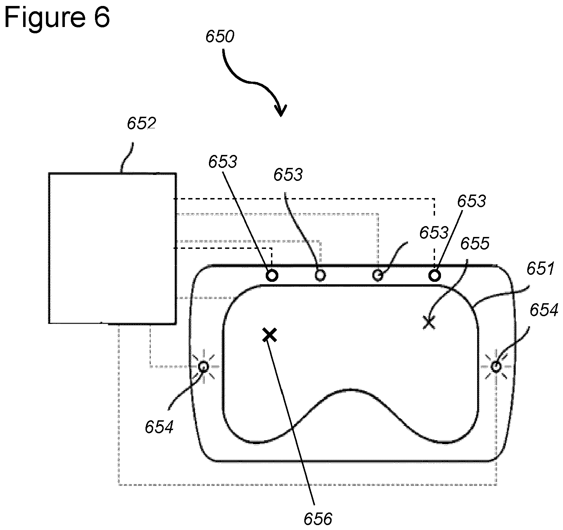

Figure 6 shows an example embodiment of a head-mounted eye tracking system 650 according to the present disclosure.

The eye tracking system 650 includes a 3D display screen 651 and a controller 652. The 3D display screen 651 may comprise one or more display screens. For example, the 3D display screen 651 may be a stereoscopic display that comprises separate display screens for each eye. The eye tracking system 650 also includes one or more cameras 653 for capturing images of the user's eyes while the user looks at the 3D display screen 651. The eye tracking system 650 can also include one or more illuminators 654. The illuminators 654 can simply be for illuminating the user's eyes such that the camera 653 can capture images of the eyes with an acceptable quality. Alternatively, the illuminators 654 can be for causing corresponding glints to be visible in the captured images such that a gaze angle and / or a 3-dimensional position of the cornea can be determined (as discussed above).

The controller 652 provides control signals to the cameras 653, the illuminators 654 and the 3D display screen 651. The controller 652 also processes images captured by the camera 653 in order to determine a gaze convergence function for a user of the eye tracking system 650 as part of a calibration routine, as will now be discussed.

The controller 652 sends a control signal to the 3D display screen 651 to cause it to present a first calibration object 655 to the user. The first calibration object 655 is presented to the user such that it appears to be at a known first object distance from the user. In any known way, the controller 652 then instructs the user of the eye tracking system 650 to look at the first calibration object 655. Such an instruction can be provided to the user visually (by presenting an appropriate instruction on the 3D display screen 651) or audibly using a speaker (not shown), for example.

While the first calibration object 655 is being displayed, and when the user should be looking at it (assuming that they are the following instructions they are presented with), one or more of the cameras 653 captures a calibration image of the user's left eye. The controller 652 then processes the captured calibration image(s) to determine a first left pupil centre value. The first left pupil centre value represents the centre of the pupil of the user's left eye as a 3-dimensional coordinate. Various examples of how images can be captured that enable the calculation of the pupil centre in three dimensions will be described below.

Also while the first calibration object 655 is being displayed, and when the user should be looking at it, one or more of the cameras 653 capture a calibration image of the user's right eye. The controller 652 then processes the calibration image(s) to determine a first right pupil centre value, which represents the centre of the pupil of the user's right eye as a 3-dimensional coordinate (preferably in the same coordinate system as the first left pupil centre value for computational efficiency of the subsequent processing steps that will be performed on the pupil centre values).

The controller 652 can then calculate a first interpupillary distance, IPD, value as the (Euclidean) distance between the first left pupil centre value and the first right pupil centre value. The calculated first IPD value and the associated first object distance will be used as a set of calibration data for determining the gaze convergence function.

We will now describe how the controller acquires another set of calibration data for a different calibration object.

The controller 652 sends a control signal to the 3D display screen 651 to cause it to present a second calibration object 656 to the user. The second calibration object 656 is presented to the user such that it appears to be at a known second object distance from the user. The second object distance is different to the first object distance for the first calibration object 655. The controller 652 then instructs the user to look at the second calibration object 656.

In the same way as discussed above, while the second calibration object is being displayed (and the user should be looking at it):

- one or more of the cameras 653 capture a calibration image of the user's left eye;
- the controller 652 determines a second left pupil centre value, which represents the centre of the pupil of the user's left eye as a 3-dimensional coordinate, based on the captured calibration image(s) of the user's left eye;
- one or more of the cameras 653 capture a calibration image of the user's right eye; and
- the controller 652 determines a second right pupil centre value, which represents the centre of the pupil of the user's right eye as a 3-dimensional coordinate (in the same coordinate system), based on the captured calibration image(s) of the user's right eye;
- the controller 652 calculates a second IPD value as the distance between the second left pupil centre value and the second right pupil centre value.

The calculated second IPD value and the associated second object distance is another set of calibration data, which is different from the first set.

The controller 652 now has two sets of calibration data for determining the gaze convergence function. It will be appreciated that one or more further sets of calibration data can be determined in the same way by presenting one or more further calibration objects to the user.

The controller then determines a gaze convergence function, which defines a relationship between IPD values and gaze convergence distances based on: i) the first IPD value and the associated first object distance; ii) the second IPD value and the associated second object distance; and iii) any further IPD values and the associated further object distances. For example, a linear curve fitting algorithm can be applied to the sets of calibration data to determine the gaze convergence function. It will be appreciated that use of a relatively high number of sets of calibration data can improve the accuracy of the gaze convergence function. However, there is a trade-off between: i) the achievable accuracy; and ii) the amount of time and computing power that is required to determine the gaze convergence function. With more points, both the time to collect the data and processing time increases.

It is advantageous to use 3-dimensional coordinates for the centres of the pupils when calculating the IPD that is used for determining the gaze convergence function. This is because any movement of the eye tracking system 650 relative to the user's eyes does not affect the calculation of the IPD. For example, a head-mounted eye tracking system can slip on the user's face such that it is in a different position with respect to the user's eyes when different sets of calibration are acquired, or when the determined gaze convergence function is subsequently applied after calibration. By using the 3-dimensional coordinates for the centres of the pupils, the likelihood that such slippage will negatively affect the accuracy of the gaze convergence function is reduced.

One example of determining a pupil centre in three dimensions includes simultaneously capturing at least two images of the eye. In such an example, a first camera 653 captures a first calibration image and, simultaneously, a second (different) camera 653 captures a second calibration image of the user's eye. Therefore, the first and second calibration images are from different angles with respect to the user's eye. The controller 652 then determines the pupil centre value, which represents the centre of the pupil of the eye as a 3-dimensional coordinate, based on the first and second calibration images. As is well-known in the art, the positions of the first and second cameras with respect to each other can be known, such that the controller 652 determines the pupil centre value as a 3-dimensional coordinate.

Another example of determining a pupil centre in three dimensions includes capturing at least one image of the eye and using reflections from a plurality of illuminators 654 (i.e., glints) that are visible in the captured image. In such an example, only one camera may be required for each eye. This includes an implementation where a single camera is used for both eyes.

In this example, while the calibration objects are being presented (and the user should be looking at them), the controller 652 causes a plurality of illuminators 654 to illuminate the user's left eye. As discussed above, such illuminators 654 can illuminate the user's eye with near infra-red (NIR) light. The controller 652 also causes a camera 653 to capture a calibration image of the user's left eye that includes reflections of light provided by the plurality of illuminators 654 as a plurality of glints. The controller 652 then determines a 3-dimensional position of the cornea of the user's left eye based on the plurality of glints (as is known in the art). The controller 652 can then determine the left pupil centre value based on: i) the captured calibration image of the user's left eye; and ii) the determined 3-dimensional position of the cornea of the user's left eye.

Similar processing is performed for the user's right eye. That is, the controller 652 causes a plurality of illuminators 654 to illuminate the user's right eye and then causes a camera 653 to capture a calibration image of the user's right eye that includes reflections of light provided by the plurality of illuminators as a plurality of glints. The controller 652 can then determine a 3-dimensional position of the cornea of the user's right eye based on the plurality of glints, and then determine the right pupil centre value based on: i) the captured calibration image of the user's right eye; and ii) the determined 3-dimensional position of the cornea of the user's right eye.

It has been found that the linear model between the IPD and convergence distance (an example of which is shown in Figure 5) may only generalize to a certain range of degrees of gaze angle. That is, the slope parameter of the linear model can be different when the user is looking straight ahead when compared with when the user is looking in a different direction (e.g., 20 degrees upward). Therefore, one or more of the examples disclosed herein can be modified such that the calibration model incorporates determined or estimated values for the gaze angle. We will now describe two examples for implementing this functionality.

In a first example, the gaze angle (or a proxy for the gaze angle) can be directly included as part of the gaze convergence function / model. In such an example, while the first calibration object is being presented, the controller 652 determines a first gaze angle for the user. While the second calibration object is being presented, the controller 652 determines a second gaze angle for the user. The first and second gaze angles can be determined in any way that is known in the art. Then, the controller 652 determines / fits the gaze convergence function based on: i) the first IPD value, the associated first object distance, and the determined first gaze angle ; and ii) the second IPD value, the associated second object distance, and the determined second gaze angle. When compared with the earlier description of curve fitting, the sets of calibration data also include the determined gaze angles. Therefore, the determined gaze angle is an additional variable that is used when determining / fitting the gaze convergence function. In this way, the gaze convergence function defines the gaze convergence distance as a function of IPD value and gaze angle.

One implementation for this first example includes using the difference in the z-dimension between the left pupil and right pupil as a proxy for gaze angle. The z-dimension is one that is perpendicular to the general plane of the 3D display screen 651. The difference in the z-dimension of the left pupil and right pupil ($z_{diff}$) can be easily calculated as the difference between the z-components of the 3-dimensional coordinates of the left and right pupil centre values. For this method, we add the z direction differences $z_{diff}$ in the left and right pupil positions as another input variable to the calibration model:

$$convergence = p_1 * IPD + p_2 * z_{diff} + p_3$$

where:

$p_1$, $p_2$, $p_3$ are curve fitting parameters;
IPD is the calculated interpupillary distance; and

$z_{diff}$ is the difference in the z-dimension between the centres of the left pupil and right pupil, which can implicitly relate to the gaze direction of the user.

In the second example, a multi-zone calibration can be performed. In such an example, the controller 652 determines a plurality of gaze convergence functions, each gaze convergence function being applicable for different gaze angles (which can be controlled by displaying appropriate calibration objects to the user). Each of the gaze convergence functions defines the gaze convergence distance as a function of IPD value in the same way that is described above with reference to Figure 5. Each of the gaze convergence functions can be applicable for a different range of gaze angles such that the virtual plane of the VR display can effectively be divided up into different gaze zones. The range of gaze angles can be offset from each other in a horizontal plane (e.g., top, middle and bottom) and / or they can be offset from each other in a vertical plane (e.g., left, middle and right). Then for each range of gaze angles (which can also be referred to as gaze zones), the controller 652 calibrates a separate IPD-convergence model. When the plurality of gaze convergence functions of this second example are subsequently applied, the eye tracking system determines the gaze zone that the user is looking at, and then applies the gaze convergence function / calibration model that is associated with that gaze zone to determine the gaze convergence distance for the user. For example, if the user is looking at the left zone, the controller 652 uses a calibrated left zone model and a calculated IPD value to compute the convergence distance. If the user is looking at the right zone, then the controller 652 uses a calibrated right zone model and a calculated IPD value to compute the convergence distance.

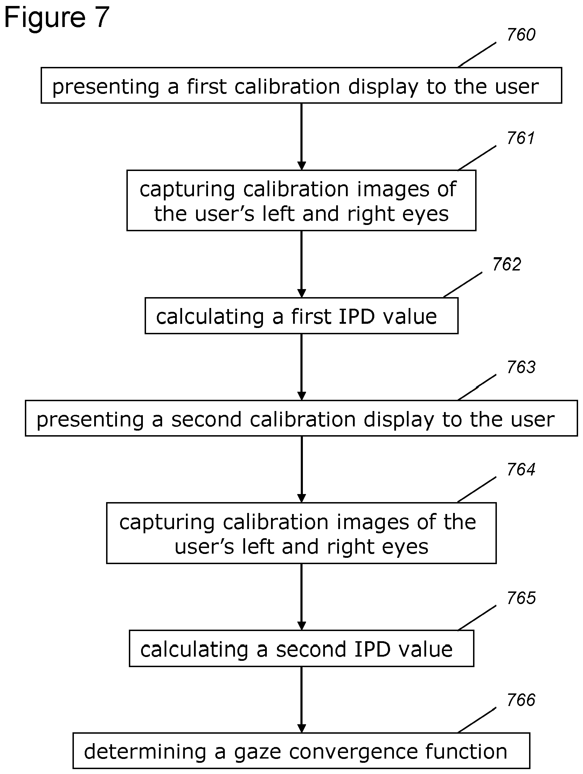

Figure 7 shows an example embodiment of a method for determining a gaze convergence function for a user of an eye tracking system.

At step 760, the method includes presenting a first calibration object to the user on one or more display screens, wherein the first calibration object appears to be at a known first object distance from the user.

While the first calibration object is being presented, the method includes at step 761: i) capturing a calibration image of the user's left eye and determining a first left pupil centre value, which represents the centre of the pupil of the user's left eye as a 3-dimensional coordinate, based on the captured calibration image of the user's left eye; and ii) capturing a calibration image of the user's right eye and determining a first right pupil centre value, which represents the centre of the pupil of the user's right eye as a 3-dimensional coordinate, based on the captured calibration image of the user's right eye.

Then, at step 762, the method includes calculating a first interpupillary distance, IPD, value as the distance between the first left pupil centre value and the first right pupil centre value (i.e., when the user is looking at the first calibration object).

At step 763, the method includes presenting a second calibration object to the user on one or more display screens, wherein the second calibration object appears to be at a known second object distance from the user. While the second calibration object is being presented, the method includes at step 764: i) capturing a calibration image of the user's left eye and determining a second left pupil centre value, which represents the centre of the pupil of the user's left eye as a 3-dimensional coordinate, based on the captured calibration image of the user's left eye; and ii) capturing a calibration image of the user's right eye and determining a second right pupil centre value, which represents the centre of the pupil of the user's right eye as a 3-dimensional coordinate (in the same coordinate system), based on the captured calibration image of the user's right eye.

At step 765, the method includes calculating a second IPD value as the distance between the second left pupil centre value and the second right pupil centre value (i.e., when the user is looking at the second calibration object).

Then, at step 766, the method determines a gaze convergence function that defines a relationship between IPD values and gaze convergence distances based on: i) the first IPD value and the associated first object distance; and ii) the second IPD value and the associated second object distance.

Advantageously IPD measurements of the present disclosure can be based on relatively stable pupil signals, such as entrance pupil and "stereo pupils". Given that the pupil is shown in both images such that its position can be calculated in 3D, it can be referred to as a stereo pupil. Such pupil signals are more robust to VR headset slippage.

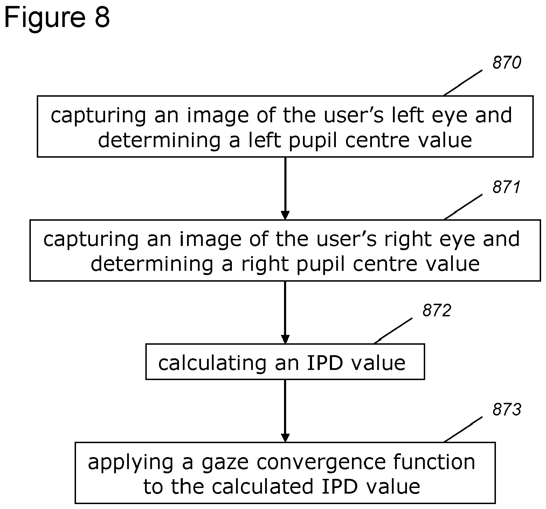

Figure 8 shows an example embodiment of a method of determining a gaze convergence distance for a user of an eye tracking system. As will be discussed below this method involves the application of a gaze convergence function, such as the one that is determined by the method of Figure 7.

At step 870, the method involves capturing an image of the user's left eye and determining a left pupil centre value. In the same way as described above, the left pupil centre value represents the centre of the pupil of the user's left eye as a 3-dimensional coordinate.

At step 871, the method involves capturing an image of the user's right eye and determining a right pupil centre value. Again, the right pupil centre value represents the centre of the pupil of the user's right eye as a 3-dimensional coordinate.

At step 872, the method calculates an interpupillary distance, IPD, value as the distance between the left pupil centre value and the right pupil centre value. Advantageously, as discussed above, calculating the IPD value using 3-dimensional coordinates for the pupil centres can mitigate against any errors that could otherwise be introduced if the alignment of the user's eye is different to when an earlier preceding calibration was performed.

Then, at step 873, the method applies a gaze convergence function to the calculated IPD value to determine a gaze convergence distance. The gaze convergence function defines a relationship between IPD values and gaze convergence distances, such as the one that is described above with reference to Figures 5 and 7.

It will be appreciated that for examples where the the gaze angle is included as part of the gaze convergence function / model, the method of Figure 8 will also include determining a gaze angle for the user such that the determined gaze angle can be used when applying the gave convergence function at step 873 to determine the gaze convergence distance.

Once the gaze convergence distance has been determined, the method can continue by using the determined gaze convergence distance to control the focal length of a varifocal lens of the eye tracking system. For example: a voltage can be applied to an electrically controlled varifocal lens; or a mechanical actuator can be activated to move the lens and change the position of the associated focal plane. In this way, the eye tracking system can be automatically controlled to improve the viewing experience of the user. Such functionality can be especially useful in extended reality systems, such as augmented and virtual reality systems.

Additionally, or alternatively, the determined gaze convergence distance can be used by the eye tracking system as part of an algorithm to more accurately identify which of a plurality of selectable objects the user is looking at. This can involve the method presenting a plurality of selectable objects to the user on one or more display screen; each of which are at a known object distance from the user and are at a known location in the one or more display screens. The method can determine a gaze angle for the user at the same time as the images of the user's left and right eyes are captured, and then use the determined gaze convergence distance in combination with the determined gaze angle to identify one of the selectable objects as a selected object. For example, a cost function can be applied that looks to minimise the differences between: i) the known object distance for each selectable object and the determined gaze convergence distance; and ii) the known location in the display screen for each selectable object and the determined gaze angle. In this way, determined gaze convergence distance is used to complement the determined gaze angle as part of an eye tracking operation.

One or more of the examples disclosed herein can provide the following advantages:

a) A more robust linear model between IPD and convergence distance, in dioptres, can be calibrated using multiple samples (two or more than two samples) from multiple depths (two or more than two depths). Using more than two samples from more than two depths can beneficially reduce the negative effects of sample noise.

b) A more robust and stable way of calculating IPD, due to the use of 3D entrance pupil based IPD measurements. This is beneficial when compared with 2D pixel-based pupil detection, which is noisier. A significant advantage is that examples disclosed herein can handle slippage issues in the system very well when compared to 2D pupil estimates.

c) A further improvement to the model by utilizing the knowledge that the linear model of IPD and convergence distance can depend on user gaze angle. There are many ways to incorporate this knowledge into the models of the present disclosure. E.g., 1. by incorporating this gaze angle in the convergence model directly and / or 2. by using the z-direction differences in the pupil positions.

d) By extending to multi-zone calibration, e.g., for each gaze zone, separate IPD-convergence models can be calibrated.

## Claims

1. A method for determining a gaze convergence function for a user of an eye tracking system, the method comprising:

   presenting a first calibration object to the user on one or more display screens, wherein the first calibration object appears to be at a known first object distance from the user;
   while the first calibration object is being presented:

      capturing a calibration image of the user's left eye and determining a first left pupil centre value, which represents the centre of the pupil of the user's left eye as a 3-dimensional coordinate, 3D, based on the captured calibration image of the user's left eye;
      capturing a calibration image of the user's right eye and determining a first right pupil centre value, which represents the centre of the pupil of the user's right eye as a 3-dimensional coordinate, based on the captured calibration image of the user's right eye;

   calculating a first interpupillary distance, IPD, value as the distance between the first left pupil centre value and the first right pupil centre value;
   presenting a second calibration object to the user on one or more display screens, wherein the second calibration object appears to be at a known second object distance from the user;
   while the second calibration object is being presented:

      capturing a calibration image of the user's left eye and determining a second left pupil centre value, which represents the centre of the pupil of the user's left eye as a 3-dimensional coordinate, based on the captured calibration image of the user's left eye; capturing a calibration image of the user's right eye and determining a second right pupil centre value, which represents the centre of the pupil of the user's right eye as a 3-dimensional coordinate, based on the captured calibration image of the user's right eye;

calculating a second IPD value as the distance between the second left pupil centre value and the second right pupil centre value; determining a gaze convergence function which defines a relationship between IPD values and gaze convergence distances based on: i) the first IPD value and the associated first object distance; and ii) the second IPD value and the associated second object distance.

2. The method of claim 1, further comprising:

presenting one or more further calibration objects to the user on one or more display screens, wherein each of the one or more further calibration objects appears to be at a known further object distance from the user; while each of the further calibration objects is being presented:

capturing a calibration image of the user's left eye and determining a further left pupil centre value, which represents the centre of the pupil of the user's left eye as a 3-dimensional coordinate, based on the captured calibration image of the user's left eye; capturing a calibration image of the user's right eye and determining a further right pupil centre value, which represents the centre of the pupil of the user's right eye as a 3-dimensional coordinate, based on the captured calibration image of the user's right eye;

calculating a further IPD value for each of the further calibration objects as the distance between the further first left pupil centre value and the further first right pupil centre value for the calibration images that were captured with the respective one or more further calibration objects being presented; and determining the gaze convergence function also based on, for each of the one or more further calibration objects: i) the further IPD value and the associated further object distance and/or wherein the gaze convergence function defines a linear relationship between: i) the inverse of IPD values; and ii) the inverse of gaze convergence distances.

3. The method of any preceding claim, wherein capturing a calibration image of the user's eye and determining a pupil centre value comprises: simultaneously capturing a first calibration image and a second calibration image of the user's eye from different cameras and determining the pupil centre value, which represents the centre of the pupil of the user's eye as a 3-dimensional coordinate, based on the first and second calibration images.

4. The method of any one of claims 1 or 2, further comprising: while the calibration objects are being presented:

illuminating the user's left eye with a plurality of illuminators and capturing a calibration image of the user's left eye that includes reflections of light provided by the plurality of illuminators as a plurality of glints, determining a 3-dimensional position of the cornea of the user's left eye based on the plurality of glints, and determining the left pupil centre value based on: i) the captured calibration image of the user's left eye; and ii) the determined 3-dimensional position of the cornea of the user's left eye; illuminating the user's right eye with a plurality of illuminators and capturing a calibration image of the user's right eye that includes reflections of light provided by the plurality of illuminators as a plurality of glints, determining a 3-dimensional position of the cornea of the user's right eye based on the plurality of glints, and determining the right pupil centre value based on: i) the captured calibration image of the user's right eye; and ii) the determined 3-dimensional position of the cornea of the user's right eye.

5. The method of any preceding claim, further comprising:

while the first calibration object is being presented, determining a first gaze angle for the user; while the second calibration object is being presented, determining a second gaze angle for the user; and wherein determining the gaze convergence function is based on: i) the first IPD value, the associated first object distance, and the determined first gaze angle; and ii) the second IPD value, the associated second object distance, and the determined second gaze angle.

6. The method of claim 5, wherein the gaze convergence function defines the gaze convergence distance as a function of IPD value and gaze angle.

7. The method of claim 5, further comprising:
determining a plurality of gaze convergence functions, each gaze convergence function being applicable for different gaze angles, wherein each of the gaze convergence functions defines the gaze convergence distance as a function of IPD value.

8. The method of claim 7, wherein each of the gaze convergence functions is applicable for a different range of gaze angles, and optionally wherein the range of gaze angles are offset from each other in a horizontal plane and / or a vertical plane.

9. The method of claim 5, further comprising:
determining a plurality of gaze convergence functions, each gaze convergence function being applicable for a different gaze zone, wherein each of the gaze convergence functions defines the gaze convergence distance as a function of IPD value.

10. The method of any preceding claim, wherein the eye tracking system is a head-mounted eye tracking system.

11. A method of determining a gaze convergence distance for a user of an eye tracking system, the method comprising:

capturing an image of the user's left eye and determining a left pupil centre value, which represents the centre of the pupil of the user's left eye as a 3-dimensional coordinate;
capturing an image of the user's right eye and determining a right pupil centre value, which represents the centre of the pupil of the user's right eye as a 3-dimensional coordinate;
calculating an interpupillary distance, IPD, value as the distance between the left pupil centre value and the right pupil centre value;
applying a gaze convergence function to the calculated IPD value to determine a gaze convergence distance, wherein the gaze convergence function defines a relationship between IPD values and gaze convergence distances.

12. The method of claim 11, further comprising:
using the determined gaze convergence distance to control the focal length of a varifocal lens of the eye tracking system.

13. The method of any one of claims 11 or 12, further comprising:

presenting a plurality of selectable objects to the user on one or more display screens, wherein the first calibration object, wherein each of the plurality of selectable objects is at a known object distance from the user and are at a known location on the on one or more display screens ;
determining a gaze angle for the user at the same time as the images of the user's left and right eyes are captured; and
using the determined gaze convergence distance in combination with the determined gaze angle to identify one of the selectable objects as a selected object.

14. The method of any one of claims 11 to 13, further comprising determining the gaze convergence function for the user of an eye tracking system, wherein determining the gaze convergence function for the user of an eye tracking system comprises:

presenting a first calibration object to the user on one or more display screens, wherein the first calibration object appears to be at a known first object distance from the user;
while the first calibration object is being presented:

capturing a calibration image of the user's left eye and determining a first left pupil centre value, which represents the centre of the pupil of the user's left eye as a 3-dimensional coordinate, based on the captured calibration image of the user's left eye;
capturing a calibration image of the user's right eye and determining a first right pupil centre value, which represents the centre of the pupil of the user's right eye as a 3-dimensional coordinate, based on the captured calibration image of the user's right eye;

calculating a first interpupillary distance, IPD, value as the distance between the first left pupil centre value and the first right pupil centre value;
presenting a second calibration object to the user on one or more display screens, wherein the second calibration object that appears to be at a known second object distance from the user;
while the second calibration object is being presented:

capturing a calibration image of the user's left eye and determining a second left pupil centre value, which represents the centre of the pupil of the user's left eye as a 3-dimensional coordinate, based on the captured calibration image of the user's left eye;
capturing a calibration image of the user's right eye and determining a second right pupil centre value, which represents the centre of the pupil of the user's right eye as a 3-dimensional coordinate, based on the captured calibration image of the user's right eye;

calculating a second IPD value as the distance between the second left pupil centre value and the second right pupil centre value;
determining a gaze convergence function based on: i) the first IPD value and the associated first object distance; and ii) the second IPD value and the associated second object distance.

15. An eye tracking system that is configured to perform the method of any preceding claim, wherein the eye tracking system is an extended reality system.

# Figure 1

# Figure 2

# Figure 3

# Figure 4

EP 4 434 439 A1

# Figure 5

Convergence range 0.12m-15m

# Figure 6

# Figure 7

*760*

presenting a first calibration display to the user

↓

*761*

capturing calibration images of the user's left and right eyes

↓

*762*

calculating a first IPD value

↓

*763*

presenting a second calibration display to the user

↓

*764*

capturing calibration images of the user's left and right eyes

↓

*765*

calculating a second IPD value

↓

*766*

determining a gaze convergence function

# Figure 8

```
                                                    870
┌─────────────────────────────────────────────┐
│  capturing an image of the user's left eye and │
│    determining a left pupil centre value        │
└─────────────────────────────────────────────┘
                        │
                        ▼                    871
┌─────────────────────────────────────────────┐
│ capturing an image of the user's right eye and │
│    determining a right pupil centre value       │
└─────────────────────────────────────────────┘
                        │
                        ▼            872
        ┌───────────────────────────┐
        │   calculating an IPD value  │
        └───────────────────────────┘
                        │
                        ▼                    873
        ┌───────────────────────────────────┐
        │  applying a gaze convergence function │
        │      to the calculated IPD value      │
        └───────────────────────────────────┘
```

Europäisches
Patentamt

European
Patent Office

Office européen
des brevets

# EUROPEAN SEARCH REPORT

**Application Number**

EP 24 16 4205

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/173206 A1 (DAS SHIULI [US] ET AL) 10 June 2021 (2021-06-10) <br> * paragraph [0129] * <br> * paragraph [0132] * <br> * paragraph [0134] - paragraph [0136] * <br> * paragraph [0139] - paragraph [0142] * <br> * paragraph [0146] - paragraph [0147] * <br> * paragraph [0152] * <br> * figures 2, 3, 5, 7A-B, 8A, 11, 14, 15 * <br> - - - - - | 1-15 | INV. <br> A61B3/113 <br> G02B27/00 <br> G02B27/01 <br> G06F3/01 |
| X | WO 2019/143844 A1 (MAGIC LEAP INC [US]) 25 July 2019 (2019-07-25) <br> * paragraph [0334] * <br> * paragraph [0336] * <br> * paragraph [0339] * <br> * paragraph [0342] - paragraph [0345] * <br> * paragraph [0378] * <br> * paragraph [0381] - paragraph [0383] * <br> * paragraph [0391] * <br> * paragraph [0399] - paragraph [0400] * <br> * paragraph [0407] - paragraph [0411] * <br> * paragraph [0415] - paragraph [0416] * <br> * paragraph [0436] - paragraph [0439] * <br> * figures 2-6, 7A, 8A, 9H * <br> - - - - - | 1-15 | |

TECHNICAL FIELDS
SEARCHED      (IPC)

A61B
G06F
G02B

The present search report has been drawn up for all claims

| Place of search | Date of completion of the search | Examiner |
|---|---|---|
| Munich | 26 July 2024 | Horváth, László |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 16 4205

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2024

| Patent document cited in search report | Publication date | Patent family member(s) | | Publication date |
|---|---|---|---|---|
| US 2021173206 A1 | 10-06-2021 | CN | 115053270 A | 13-09-2022 |
| | | EP | 4073689 A1 | 19-10-2022 |
| | | JP | 2023504207 A | 01-02-2023 |
| | | US | 2021173206 A1 | 10-06-2021 |
| | | US | 2022146823 A1 | 12-05-2022 |
| | | US | 2023139308 A1 | 04-05-2023 |
| | | US | 2023418054 A1 | 28-12-2023 |
| | | WO | 2021119212 A1 | 17-06-2021 |
| WO 2019143844 A1 | 25-07-2019 | AU | 2019209930 A1 | 09-07-2020 |
| | | CA | 3087333 A1 | 25-07-2019 |
| | | CN | 111869200 A | 30-10-2020 |
| | | EP | 3741109 A1 | 25-11-2020 |
| | | EP | 4354207 A2 | 17-04-2024 |
| | | IL | 275822 A | 31-08-2020 |
| | | IL | 305833 A | 01-11-2023 |
| | | JP | 7390297 B2 | 01-12-2023 |
| | | JP | 7488405 B2 | 21-05-2024 |
| | | JP | 2021511723 A | 06-05-2021 |
| | | JP | 2023160877 A | 02-11-2023 |
| | | JP | 2024088684 A | 02-07-2024 |
| | | KR | 20200110367 A | 23-09-2020 |
| | | US | 2019243448 A1 | 08-08-2019 |
| | | US | 2021105456 A1 | 08-04-2021 |
| | | US | 2022057862 A1 | 24-02-2022 |
| | | US | 2024108217 A1 | 04-04-2024 |
| | | WO | 2019143844 A1 | 25-07-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9041787 B
- WO 2019158709 A